# EUROPÄISCHE PATENTSCHRIFT

(11) **EP 1 994 916 B1**
(45) Veröffentlichungstag und Bekanntmachung des Hinweises auf die Patenterteilung: **14.10.2020**
(21) Anmeldenummer: 08163636.7
(22) Anmeldetag: 08.05.2003
(51) Int. Cl.: A61F 13/02, A61F 13/00, A61F 5/445

(54) **Absorptionskörper zum Anschluss an den menschlichen Körper**
Absorption bodies for attachment to the human body
Corps d'absorption destiné au raccordement au corps humain

(30) Priorität: 08.05.2002 DE 20207356 U
(43) Veröffentlichungstag der Anmeldung: 26.11.2008
(62) Teilanmeldung aus: 03727455.2
(73) Patentinhaber: BSN medical GmbH, 20253 Hamburg (DE)
(72) Erfinder: Riesinger, Birgit, 48149 Münster (DE)
(74) Vertreter: FARAGO Patentanwälte

(56) Entgegenhaltungen:
- WO-A1-00/09056
- WO-A2-98/28013
- DE-A1- 3 826 425
- DE-A1- 10 059 439
- DE-A1- 19 827 716
- GB-A- 2 099 306
- US-A- 3 895 629
- US-B1- 6 488 670

## Beschreibung

Beschrieben wird ein Absorptionskörper zum Anschluß an den menschlichen Körper, insbesondere zum Aufsaugen von Flüssigkeiten, welche aus menschlichen Körperstellen, wie Wunden, austreten, aufweisend:
- einen im wesentlichen flachen Materialabschnitt aus Absorptionsmaterial, bestehend aus einem aufsaugenden Vlies mit darin verteilten Superabsorber-Teilchen,
- und eine flüssigkeitsdurchlässige Hülle, die das Absorptionsmaterial umgibt und eine Barriere gegen feste Ausscheidungen bildet und die den Durchtritt von anderen ausgetretenen Substanzen zu dem innerhalb der Hülle angeordneten Materialabschnitt aus Absorptionsmaterial ermöglicht,
wobei die Hülle wenigstens teilweise von einer Naht umgeben ist.

Aus der DE-OS 100 59 439 der Anmelderin ist ein Absorptionskörper bekannt, welcher eine Hülle mit einem darin untergebrachtem Absorptionsmaterial aufweist. Das Absorptionsmaterial liegt in Form eines textilen Vliesmaterials auf Cellulosebasis vor, in dem eine Menge an Superabsorber-Granulat verteilt ist. Die volle Aufsaugkapazität des dicht verpackten Absorptionsmaterials kann nicht ausgenutzt werden, weil dieses dicht von der Hülle umgeben ist. Infolgedessen ist die Anzahl von benutzten Absorptionskörpern bei den Patienten relativ hoch, da diese oft ausgewechselt werden müssen. Dies betrifft beispielsweise die Kolostomie- und Ileostomie-Patienten, die einer kostspieligen, postoperativen Nachsorge unterzogen werden müssen.

Nachteilig ist auch, dass die vom Absorptionsmaterial freigesetzten Superabsorber-Teilchen durch die Hülle - im nicht benutzten Zustand des Absorptionskörpers - nach außen gelangen können. Dies erschwert den Sterilisationsprozess des Absorptionskörpers.

Ein weiterer Nachteil des bekannten Absorptionskörpers ist, dass die umlaufende Schweißnaht den Absorptionskörper an seinem Rand versteift und ungünstig auf das Hautgewebe des Patienten, beispielsweise auf gequollene, entzündete Wundränder einwirken kann.

Die Aufgabe der Erfindung ist, einen verbesserten, kostengünstigen Absorptionskörper der eingangs genannten Art anzugeben, der über die Möglichkeit verfügt, eine volle Aufsaugkapazität des Absorptionsmaterials auszunutzen.

Diese Aufgabe ist durch die kennzeichnenden Merkmale des Anspruchs 1 gelöst.

Die im Oberbegriff angedeuteten Körperstellen beziehen sich im allgemeinen auf die Haut-, Schleimhaut- und/oder Wundoberflächen.

Vorzugsweise weist die Hülle eine kleinmaschige Struktur auf, deren Poren zwischen 0,05 mm bis 1,0 mm, vorzugsweise zwischen 0,20 mm bis 0,50 mm liegen. Damit sind die Poren jeweils kleiner als die unbenetzten Superabsorber-Teilchen, die innerhalb der Hülle räumlich verbleiben können.

Die Poren oder Mischen können durch die Fäden- oder Faserabschnitte begrenzt sein, welche im Schnitt durch die Hülle etwa bogenförmig sind und mit .ihren Bogen-Scheiteln nach außen zeigen.

Vorzugsweise ist die Hülle aus einem Textilmaterial von sehr weichem Griff hergestellt, beispielsweise aus synthetischem, hauchdünnem, gesundheitsunbedenklichem Vliesstoff. Die weiche Struktur des Materials trägt zu einer schonenden Behandlung der Wunde bei, da sich die freien Kanten am Überstand der Hülle beim Kontakt mit der Wunde verformen bzw. biegen können. Als Material für die Hülle eignen sich insbesondere perforierte bzw. maschenartige, hydrophobe, vliesartige Polyolefin-Folien, wie Polyethylen- oder Polypropylen-Folie, oder ein Naturstoff-Gewebe oder -vlies, das mit hautverträglichen Substanzen, wie Silicon, Paraffin, Wachs und dgl. imprägniert ist. Vorzugsweise ist das Material der Hülle wenigstens monoaxial dehnbar.

Das innerhalb der Hülle angeordnete Absorptionsmaterial kann aus einem Kunststoff-Gewebe oder -vlies, beispielsweise aus Zellstoffasern gebildet sein.

Der an sich bekannte, sogenannte Superabsorber kann beispielsweise in Form eines Granulats auf Basis von Natriumacrylat-Acrylsäure-Polymerisat vorliegen, das in der Trägermatrix, d.h. im celluloseartigen Materialabschnitt feinverteilt ist.

Das Absorptionsmaterial kann auch gel- oder schwammartig sein. Es ist auch denkbar, als Absorptionsmaterial ein flächenhaftes, schwammartiges Gebilde zu verwenden, das offenporig und mit einem Hydrogel durchsetzt ist. Das Absorptionsmaterial, darin Hydrogel kann mit wundheilungfördernden Materialien angereichert sein, beispielsweise mit lytischen Enzymen aus der Gruppe von Proteasen oder mit Peptiden und/pder mit Antibiotika. Hierbei empfiehlt sich, zur Herstellung des Hydrogels ein Heteropolysaccharid, wie Agar-Agar, oder andere bioverträgliche Substanzen zu verwenden.

Der flächenhafte Absorptionskörper kann oval, kreisrund, polygonal bzw. trapezförmig sein.

Vorzugsweise ist die Fläche des Materialabschnittes um 20% bis 35% kleiner als die Fläche der Hülle, wobei die letztere durch die umlaufende Naht begrenzt ist.

Von großem Vorteil ist, dass die Hülle in Draufsicht auf seine Flachseite einen umlaufenden, über die Naht hinausragenden Überstand aufweist, so dass der Absorptionskörper frei von harten, scharfen Kanten und Ecken ist und die Verletzung von Gewebe des Patienten in jedem Moment des Absorptionsvorgangs weitgehend vermieden werden kann.

Am Überstand kann eine Vielzahl von Einschnitten eingearbeitet sein, die vorzugsweise etwas kürzer als die Breite des Überstands sind. Diese Maßnahme trägt dazu bei, dass die äußeren Kanten des Überstandes noch mehr nachgeben können. Weiterhin kann der Überstand und/oder die umlaufende Naht in den Eckbereichen des Absorptionskörpers abgerundet sein.

Das Absorptionsmaterial kann aus zwei oder mehr innerhalb der Hülle angeordneten, flach liegenden Lagen bestehen, die gleiche oder unterschiedliche Saugkraft aufweisen. Weiterhin kann zwischen den Lagen eine Innenwand angeordnet sein, die die Lagen voneinander vollständig oder teilweise trennt.

Sowohl die Hülle, als auch das innerhalb der Hülle liegende Absorptionsmaterial kann mit einem geruchshemmenden und/oder -neutralisierenden bzw. -maskierenden Zusatz versehen sein, beispielweise mit Aktivkohlefilter oder mit Zusätzen, mit denen aktiv z.B. Stickstoff- oder Schwefel-Wasserstoff-Verbindungen aus dem gasförmigen Milieu entfernt werden können.

Als geruchshemmende können Naturstoffe gelten, bzw. Extrakte daraus, bioinerte Materialien sowie Kunststoffe, die eine bestimmte Elektronegativität aufweisen, wodurch eine Anlagerung und Bindung von Schwefel-Wasserstoff-Verbindungen erfolgen kann. Hierbei weisen die Kunststoffmoleküle bestimmte geometrische Formen auf, wie Prismen oder Spiralen.

Insgesamt ist das so ausgelegte Absorptiohsmaterial, sowie die oben beschriebene Hülle in der Lage, ein inneres, wundheilungsförderndes Mikroklima zu erzeugen.

Zwischen dem Absorptionsmaterial und der Hülle kann eine zusätzliche, innere Umhüllung aus einem saugfähigen Material, wie Baumwolle oder Zellstoff, angeordnet sein, dessen Saugfähigkeit gering bzw. kleiner als die des eigentlichen Absorptionsmaterials ist. Eine solche innere Umhüllung erschwert den direkten Kontakt zwischen den Schleimhautzellen an der Wunde oder Körperhöhle und dem Absorptionsmaterial. Anstelle der inneren Umhüllung kann zu demselben Zweck zwischen dem Absorptionsmaterial und der Hülle eine.Zwischenlage in Form eines saugfähigen Materialabschnittes aus Baumwolle, Zellstoff oder aus Kunstfasern angeordnet sein.

An dieser Zwischenlage kann wenigstens eine Öffnung oder Aussparung eingearbeitet sein, so dass beim Gebrauch die Sekrete teils durch die Zwischenlage aufgesaugt werden und teils über die Öffnung direkt in den Superabsorber gelangen. Auf dieser Weise wird erreicht, dass die Sekrete zumindest teilweise kontrolliert verteilt werden können.

Anstelle der Zwischenlage von begrenzter Saugfähigkeit kann eine flüssigkeitsundurchlässige Folie verwendet werden, die ebenfalls mit wenigstens einer Öffnung versehen ist. In diesem Fall empfehlt sich, die Folie mit der Hülle zu verkleben.

Das Absorptionsmaterial kann aus zwei oder mehr innerhalb der Hülle angeordneten, flach liegenden Lagen bestehen, die gleiche oder unterschiedliche Saugkraft aufweisen. Weiterhin kann zwischen den Lagen eine Innenwand angeordnet sein, die die Lagen voneinander vollständig oder teilweise trennt.

Weiterhin kann an wenigstens einer Außenseite der flüssigkeitsdurchlässigen Hülle teils oder ganzflächig ein flüssigkeitsdurchlässiger Folienabschnitt angebracht sein.

Die Hülle und/oder das Absorptionsmaterial kann an ihrem/seinem Umfang mit einer an den Körper des Patienten haftenden Substanz, wie Klebstoff, versehen sein. Als Klebstoff eignen sich beispielsweise Pektin-Cellulose-Verbindungen. Die abzudichtende Substanz soll allerdings ein einfaches Entfernen bzw. Abziehen des Absorptionskörpers vom Körper des Patienten ermöglichen. Ein solcher Absorptionskörper ist insbesondere zum Auflegen auf die Wunde und zur direkten Behandlung bei postoperativen Drainagesystemen geeignet. Vorzugsweise ist diese Substanz so gewählt, dass sie ein einfaches Abziehen des Absorptionskörpers aus dem Körper des Patienten erlaubt.

Der Absorptionskörper gemäß Erfindung kann im Rahmen der Wundversorgung, insbesondere bei der sekundären Wundheilung, d. h. bei starker Exudation und bei Transsudation, beispielsweise bei Decubitus, sekundär heilenden Laparotomie-Wunden, ulcus cruris und bei postoperativen Wunden oder Nähten verwendet werden.

Weitere Merkmale und Vorteile der Erfindung sind den nachstehenden Ausführungsbeispielen zu entnehmen, die anhand der Zeichnung näher erläutert sind. Die Figuren der Zeichnung zeigen im einzelnen:
- Fig.1: einen rechteckigen Absorptionskörper in Draufsicht auf seine Flachseite;
- Fig. 2: einen elliptischen Absorptionskörper, ebenso in Draufsicht auf seine Flachseite;
- Figuren 3a und 3b: Teil des Absorptionskörpers in einem Schnitt A-A gemäß Fig.1;
- Fig. 4: einen trapezförmigen Absorptionskörper;
- Fig. 5: einen quadratischen Absorptionskörper in Draufsicht auf seine Flachseite;
- Fig. 6: eine weitere Ausführungsform des Absorptionskörpers;
- Fig. 7: einen Absorptionskörper mit Absorptionsmaterial in Form eines mehrdimensionalen Körpers, in einer perspektivischen Sicht;
- Fig. 8a: einen Absorptionskörper mit Flügeln, in Draufsicht auf seine Flachseite gesehen;
- Fig. 8b: den Absorptionskörper gemäß Fig. 8a in einer perspektivischen Sicht;
- Figuren 9 bis 13: andere Ausführungsformen des Absorptionskörpers, in einem schematischen Schnitt;
- Fig. 14: einen rechteckigen Absorptionskörper mit Enschnitten am Überstand, in Draufsicht auf seine Flachseite;
- Figuren 15 und 16: den Absorptionskörper gemäß Fig. 12 mit einer inneren Öffnung, in Draufsicht auf seine Flachseite und im Schnitt;
- Fig. 17: den Absorptionskörper gemäß Fig. 1 beim Abstreifen aus einem Ileostomiebeutel, in einer perspektivischen Ansicht;
- Fig. 18: einen trapezförmigen Absorptionskörper gemäß der Fig. 4, eingelegt in einen Kolostomiebeutel, in einer schematischen Draufsicht auf die Flachseite des Beutels;
- Fig. 19: einen Teilschnitt durch den Absorptionskörper im Nahtbereich;
- Fig. 20: ein Ensemble, bestehend aus drei Absorptionskörpern;
- Fig. 21: ein Ensemble, bestehend aus zwei miteinander über eine abreißbare Perforationslinie verbundenen Absorptionskörpern; und
- Fig. 22: einen rechteckigen Absorptionskörper mit abgerundeten Eckbereichen, in Draufsicht auf seine Flachseite.

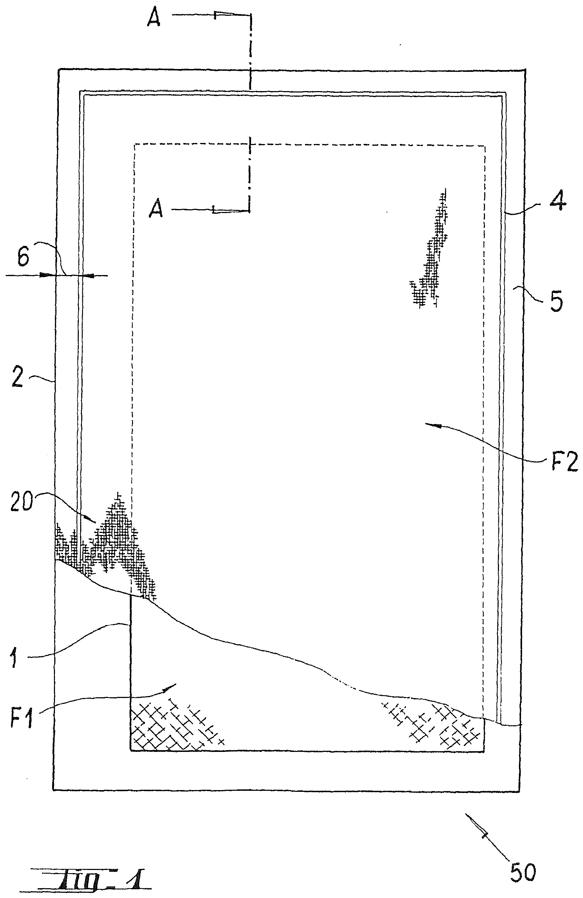

In Fig. 1 ist ein Absorptionskörper 50 dargestellt, der aus einer Hülle 2 und einem darin lose untergebrachten Absorptionsmaterial 1 in Form eines flachen, zelloloseartigen Materialabschnittes besteht. Der Materialabschnitt weist eine Dicke etwa 2 mm auf und ist mit einem Superabsorber-Granulat durchsetzt. Die Ausmaße des Materialabschnittes betragen:

| | | |
|---|---|---|
| - | Länge: | 15,5 cm; |
| - | Breite: | 8,0 cm; |
| - | Dicke: | 2,0 mm |

Die Hülle 2 gemäß Fig. 1 ist aus zwei gleichen, an ihrer Peripherie miteinander über eine Schweißnaht 4 verbundenen Seitenwänden 2.1, 2.2 gefertigt. Die Ausmaße der Hülle 2 betragen:

| | | |
|---|---|---|
| - | Länge: | 18 cm; |
| - | Breite: | 10 cm, |

bemessen zwischen den gegenüberliegenden Nähten.

Daraus resultiert, dass die Fläche F1 etwa 2/3 der Fläche F2 ausmacht.

Durch die vorteilhaften geometrischen Verhältnisse, hier: durch die Unterschiede zwischen den Flächen F1 und F2 der Hülle 2 und des Absorptionsmaterials 1, kann sich das Aufsaugvermögen des Absorptionmaterials voll entfalten.

Die Hülle 2 weist eine kleinmaschige Struktur auf, deren Poren 20 im vorliegenden Fall etwa 0,15 mm bis 0,25 mm groß sind.

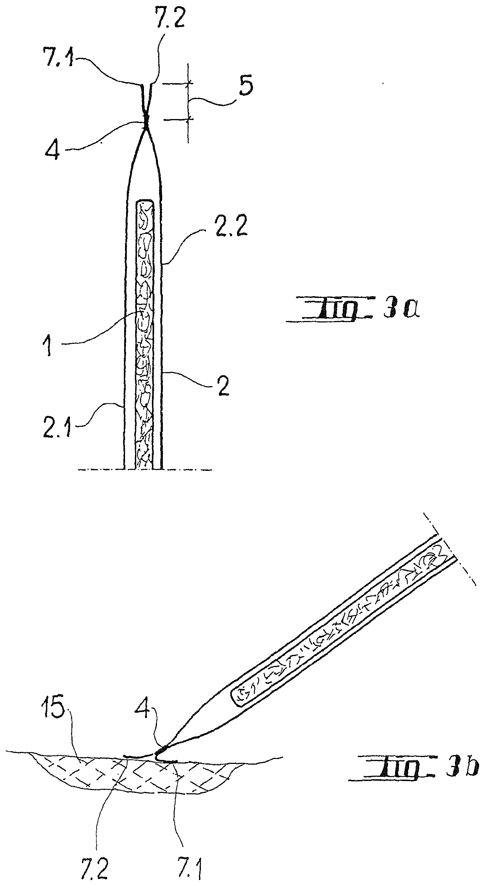

Eine Besonderheit des Absorptionskörpers 50 ist ein umlaufender, über die Schweißnaht 4 hinausragender Überstand 5, dessen Breite 6 (vgl. Fig. 1) zwischen 4 mm und 5 mm liegt. Die Aufgabe des Überstands 5 ist, eine sanfte Pufferzone zwischen dem Gewebe des Patenten, wie offene Wunde 15 (vgl. Fig. 3b), und der Schweißnaht 4 zu bilden, wenn mit dem Absorptionskörper unvorsichtig manipuliert wird, d.h. wenn der Absorptionskörper mit seiner Kante bzw. Ecke die schmerzhafte Wunde berührt. Die Figuren 3a und 3b zeigen, dass die freien Außenkanten 7.1 und 7.2 der Hülle am Überstand 5 keinerlei Versteifungen aufweisen. Vielmehr verformen sich die Außenkanten 7.1 und 7.2 so, wie in Fig. 3b dargestellt. Die Außenkanten 7.1 und 7.2 gehen auseinander oder weichen sanft in eine Richtung ab, so dass die Wunde 15 vor der Berührung mit der Naht (insbesondere bei hochkant oder schräg gestellten Absorptionskörper) geschützt wird.

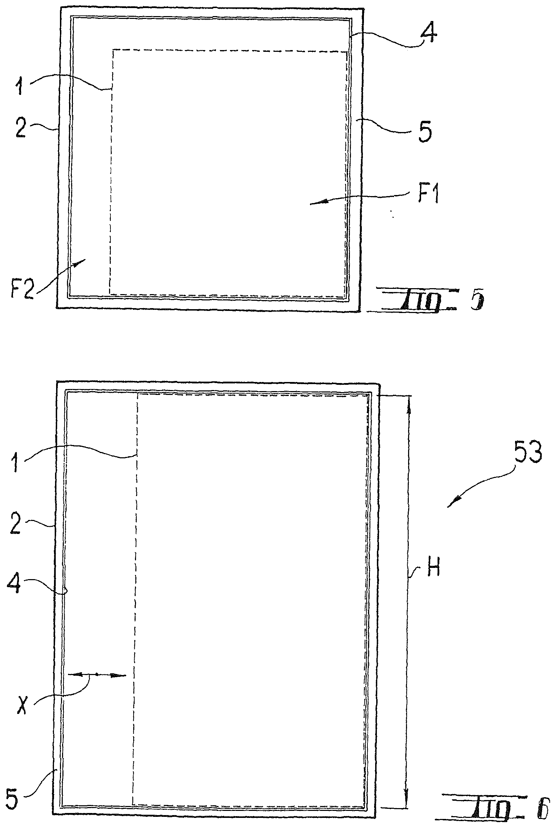

Es ist vorgesehen, auch andere, insbesondere quadratische Hüllen mit Überstand 5, beispielsweise von Ausmaßen 5,0 cm x 5,0 cm oder 7,5 cm x 7,5 cm zu verwenden, bei denen die Fläche F1 des inneren, saugfähigen Materialabschnittes entsprechend kleiner ist (vgl. Fig. 5).

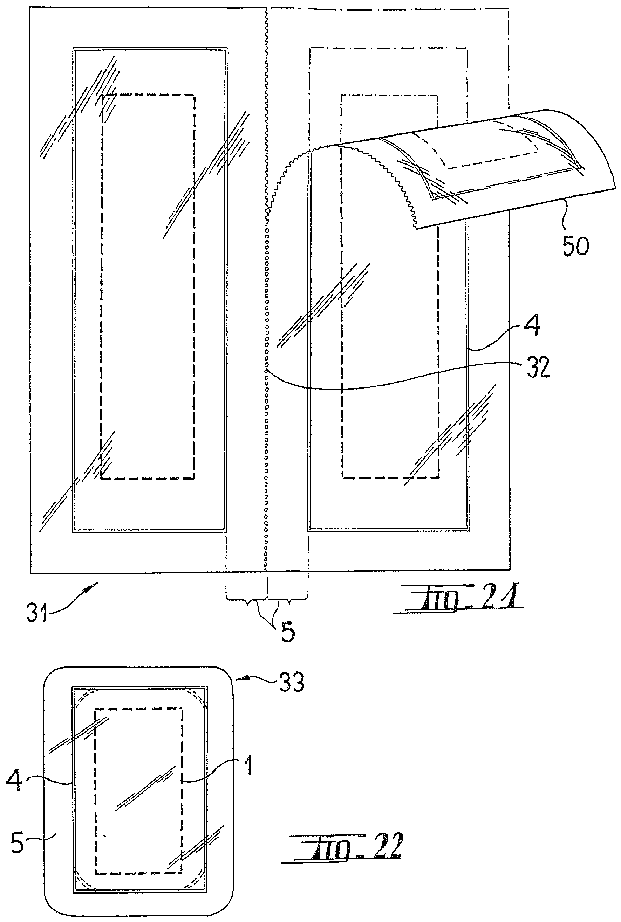

Die Fig. 22 zeigt einen flächenhaften Absorptionskörper, dessen Überstand 5 an den Eckbereichen 33 sanft abgerundet ist. Mit Strichlinien sind Abrundungen der Naht 4 in den Eckbereichen 33 gezeigt.

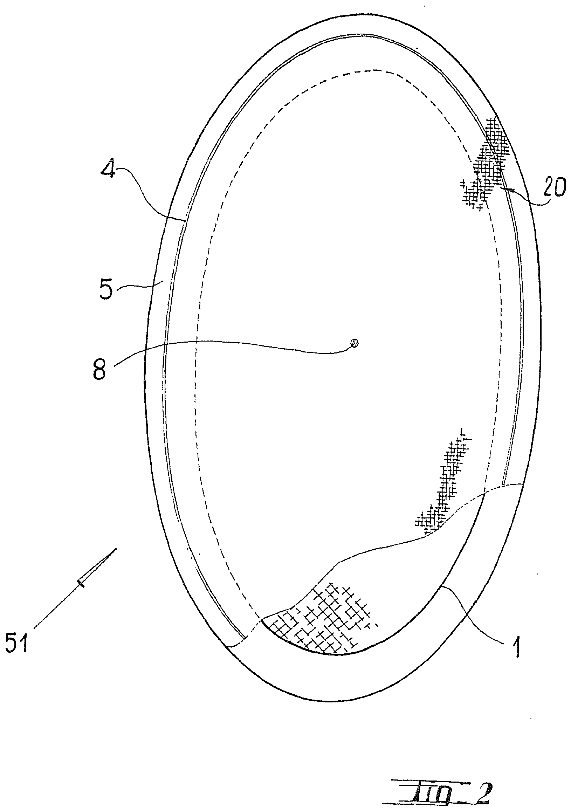

In Fig.2 ist ein elliptischer Absorptionskörper 51 dargestellt, bei dem das Absorptionsmaterial 1 über einen einzigen, mittig angeordneten Klebepunkt 8 an der Hülle 2 fixiert ist. Das Absorptionsmaterial ist von der Hülle 2 nicht trennbar. Als Klebstoff kann auch eine gesundheitsunbedenkliche Klebesubstanz oder -mischung verwendet werden, die sich beim Aufquellen des Absorptionsmaterials auflöst (nicht dargestellt).

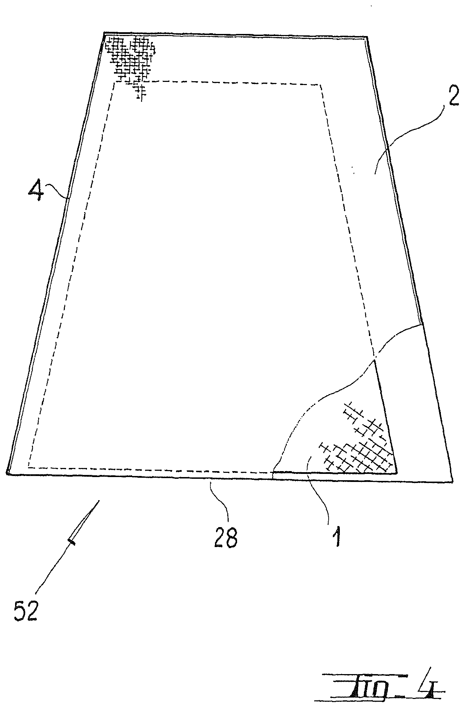

Die Fig. 4 zeigt einen trapezförmigen Absorptionskörper 52, in dessen Hülle 2 lose ein Abschnitt des bereits beschriebenen textilen Absorptionsmaterials mit Superabsorber-Teilchen platziert ist. Die einstückig hergestellte Hülle 2 ist bei ihrer Trapez-Basiskante 28 zusammengefaltet und an den übrigen Kanten zusammengeschweißt. Im vorliegenden Fall weist die Hülle keinen über die Schweißnaht hinausragenden Überstand auf. Da die Fläche F1 des textilen Absorptionsmaterials wesentlich- kleiner als die Fläche F2 der Hülle ist, kann der Absorptionsvorgang unbehindert verkaufen.

Der Fig. 6 ist eine abweichende Ausführungsform des Absorptionskörpers (Bezugszahl 53) zu entnehmen, bei der das Höhenmaß (H) des rechteckigen Materialabschnittes dem Abstand zwischen zwei gegenüberliegenden Nähten entspricht und bei der die Breite der Hülle größer als die Breite des Materialabschnittes ist. Die Verschiebbarkeit des inneren Materialabschnittes ist nur in einer Richtung X gesichert.

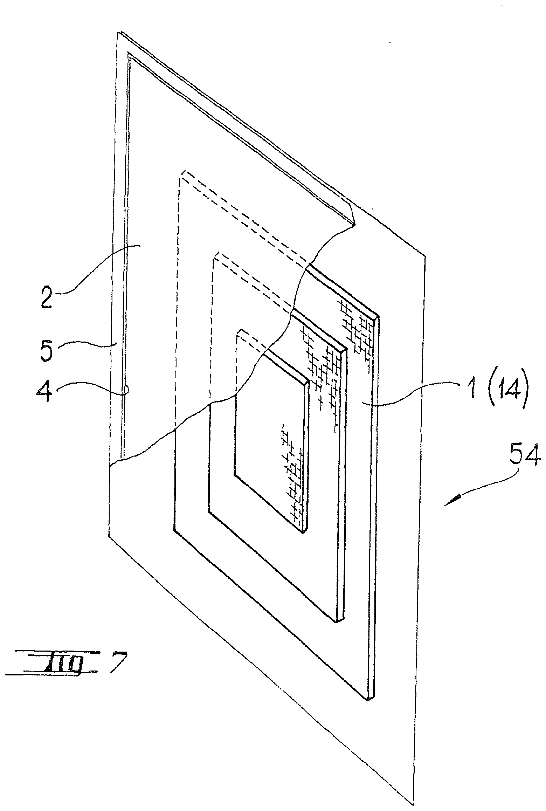

Die Fig. 7 zeigt, dass das freibewegliche Absorptionsmaterial 1 anstelle des flachen in Form eines prismatischen Körpers 14 vorliegen kann, der an jeweilige Erhabenheit und/oder Vertiefung der Wunde angepasst werden kann. Der Körper 14 besteht aus mehreren, treppenpyramidartig aufeinander gelegten und miteinander wenigstens punktweise verschweißten, saugfähigen Materialabschnitten. Diese Materialabschnitte können gleiche oder unterschiedliche Saugfähigkeit aufweisen. Es sind beliebige prismatische Formen des freibeweglichen Absorptionsmaterials 1 denkbar. Insgesamt ist der in Fig. 7 dargestellte Absorptionskörper 54 ebenfalls flach, jedoch etwas dicker als die einzelnen Absqrptionskörper 50; 51; 52 und 53.

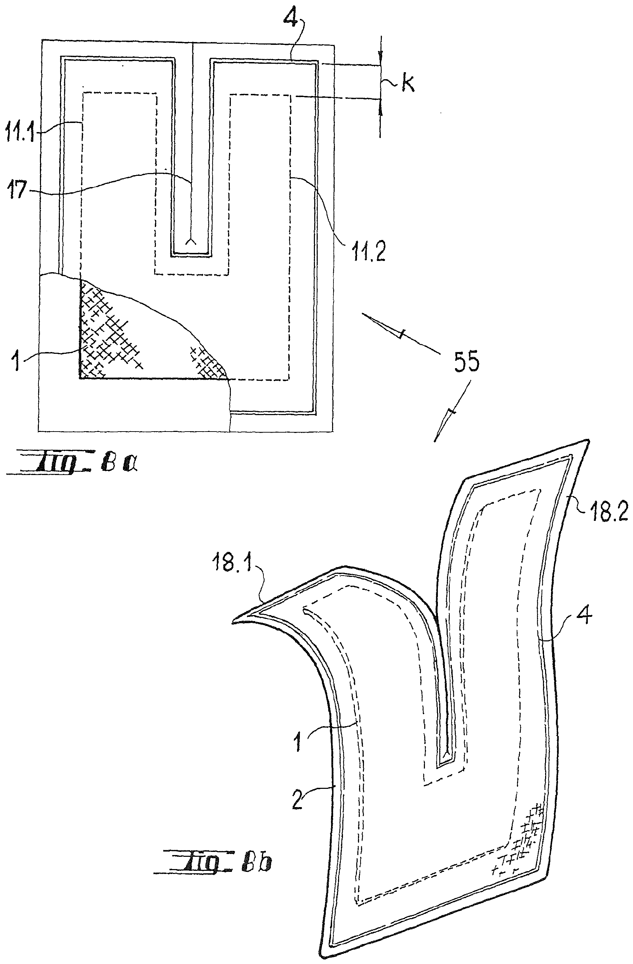

Bei einer anderen, Ausführungsform (Bezugszeichen 55; vgl. Figuren 8a und 8b) handelt sich um einen Absorptionskörper, dessen Hülle 2 eine bis zur Mitte hin reichende Schnittlinie 17 aufweist, die senkrecht gegenüber der Naht 4 bzw. dem Überstand angeordnet ist und ein Teil des Absorptionskörpers in zwei biegsame Flügel 18.1, 18.2 teilt.
Durch die flügelartige Ausbildung kann der Absorptionskörper um eine Drainage, bzw. schlauchartige Austrittsstelle gelegt werden. Der absorbierende Materialabschnitt 1 ist im vorliegenden Fall U-förmig ausgebildet, wobei seine beide U-Schenkel 11.1, 11.2 jeweils völlig durch die Naht und den Überstand umgeben sind. Anstelle der Schnittlinie kann eine U- oder V-förmige Aussparung (nicht dargestellt) vorgenommen werden.

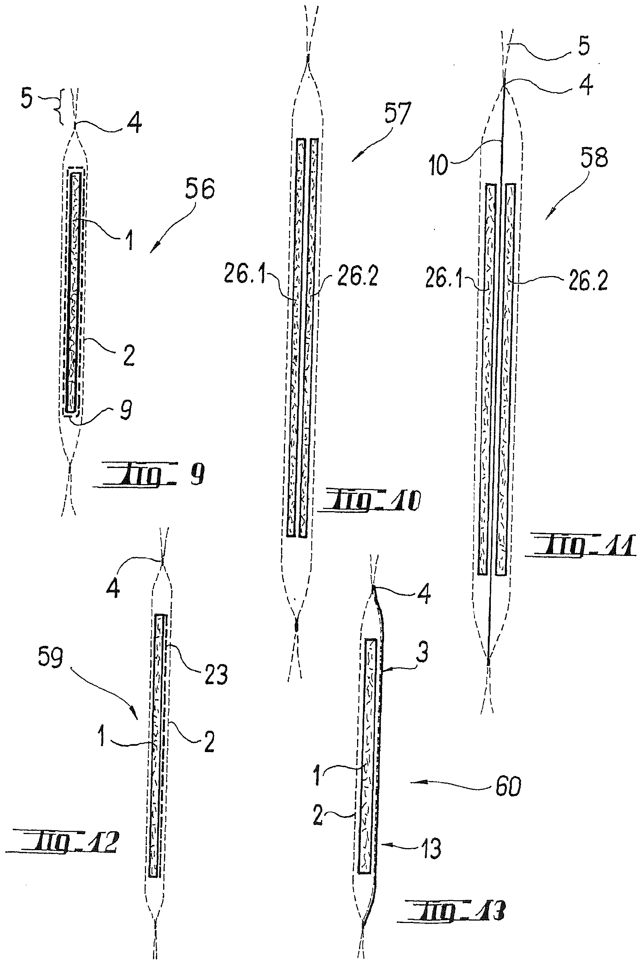

In der Fig. 9 ist ein Absorptionskörper 56 mit einer inneren Umhüllung 9 dargestellt, die das Absorptionsmaterial 1 (Superabsorber) vollständig umgibt. Die Umhüllung 9 ist aus einem flüssigkeitsabsorbierenden Material gefertigt, dessen Saugfähigkeit jedoch kleiner als die des inneren Absorptionskörpers 1 ist. Die Umhüllung 9 ist wiederum von der äußeren Hülle 2 mit Überstand 5 umgeben.

Die Fig. 10 zeigt einen Absorptionskörper (Bezugszahl 57), bei dem das Absorptionsmaterial aus zwei Lagen 26.1, 26.2 26.1, 26.2 eines Superabsorbers besteht, wobei sich diese von der Saugkraft her unterscheiden.

Bei einer ähnlichen Ausführungsform (vgl. Fig. 11) handelt sich um einen Absorptionskörper 58, welcher über eine flüssigkeitsundurchlässige Trennwand 10 verfügt, die den Absorptionskörper in zwei gleiche Kammern 21.1, 21.2 unterteilt. In jeder Kammer 21.1, 21.2 ist eine Lage 26.1; 26.2 des Absorptionsmaterials untergebracht.

In der Fig. 12 ist ein Absorptionskörper 59 mit einer inneren Zwischenlage 23 dargestellt. Die aus Baumwolle bestehende Zwischenlage 23 weist eine kleinere Saugfähigkeit als die des eigentlichen Absorptionsmaterials 1 auf und hat die Aufgabe, den direkten Kontakt zwischen den Schleimhautzellen an der Wunde und dem Absorptionsmaterial zu erschweren.

Wie in der Fig. 13 abgebildet, ist an einer Außenseite 13 eines Absorptionskörpers 60 ein flüssigkeitsundurchlässiger Folienabschnitt 3 angebracht. Der Folienabschnitt 3 ist vorzugsweise transparent und erstreckt sich über die ganze, durch die umlaufende Naht 4 begrenzte Fläche, ausgenommen Überstand 5. Zur Herstellung des Absorptionskörpers 60 eignen sich nahezu alle der vorstehend aufgeführten Ausführungsformen (außer Bezugszahl 58).

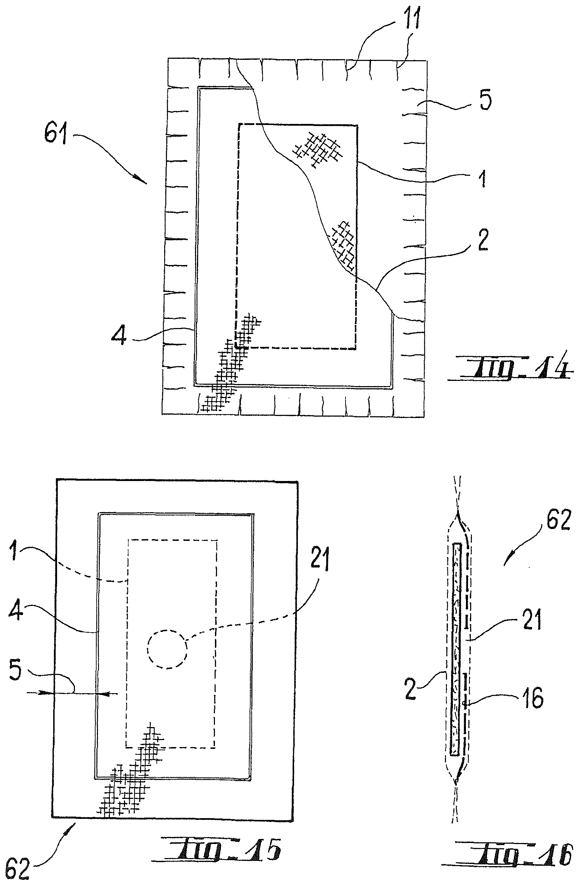

Ein in der Fig. 14 gezeigter Absorptionskörper 61 zeichnet sich durch mehrere Einschnitte 11 aus, die am Überstand 5 eingearbeitet und umlaufend an diesem angeordnet sind. So ist ein "zerfetzter" Überstand 5 entstanden, dessen äußere Kante noch mehr nachgeben kann.

In den Figuren 15 und 16 ist ein weiterer Absorptionskörper (Bezugszahl 62) gezeigt, dessen Konstruktion eine Weiterentwicklung der in der Fig. 12 dargestellten Ausführungsform ist. Eine umlaufend an der Naht 4 angebrachte Zwischenlage 16 weist eine mittige, runde Öffnung 21 auf, die die Aufgabe hat, die einfließenden Sekrete besser zu verteilen. Die aus Baumwolle bestehende Zwischenlage 16 weist ebenfalls eine kleinere Saugfähigkeit als die des eigentlichen Absorptionsmaterials 1 auf. Anstelle der saugfähigen kann eine flüssigkeitsundurchlässige, beispielsweise folienartige (nicht dargestellt) Zwischenlage mit der Öffnung 21 vorgesehen sein.

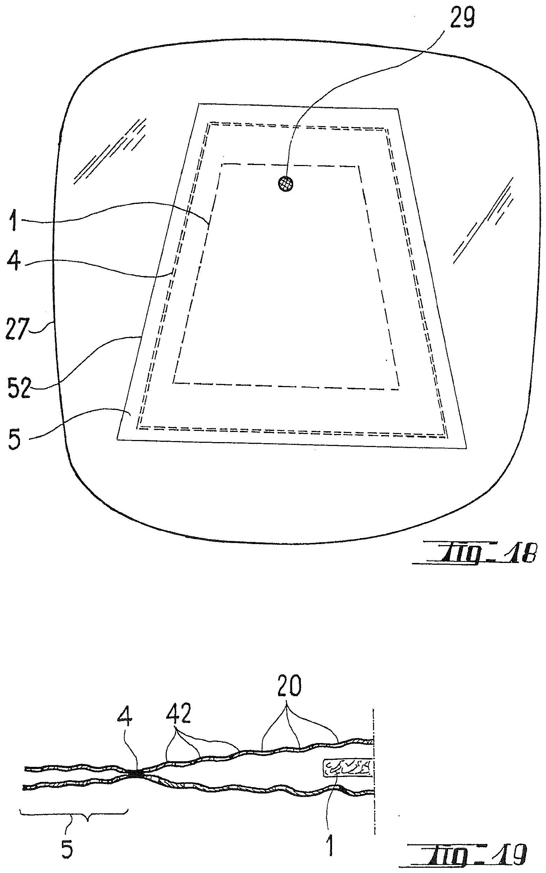

Die Fig. 18 zeigt die Anordnung eines trapezförmigen Absorptionskörpers 52 innerhalb eines schematisch angedeuteten Kolostomiebeutels 27. Der Absorptionskörper 52 ist bei 29 (knopfförmige Verklebung) mit dem Kolostomiebeutel verbunden. Zur Verklebung eignet sich am besten ein bioverträglicher Klebstoff. Die Verklebung kann lösbar sein.

In der Fig. 19 ist ein vergrößerter Teilschnitt durch die Hülle 2 im Bereich des Überstands 5 und der umlaufender Naht 4 gezeigt. Das glatte und hydrophobe Material der Hülle 2 weist zahlreiche, sanft verlaufende und nach außen gerichtete Wölbungen 42 auf, die zwischen den Poren 20 liegen. Durch diese Materialstruktur der Hülle wird erreicht, dass die Flüssigkeit über die Außenfläche der Hülle gleitet und durch die Poren einfacher ins Innere des Absorptionskörpers gelangen kann.

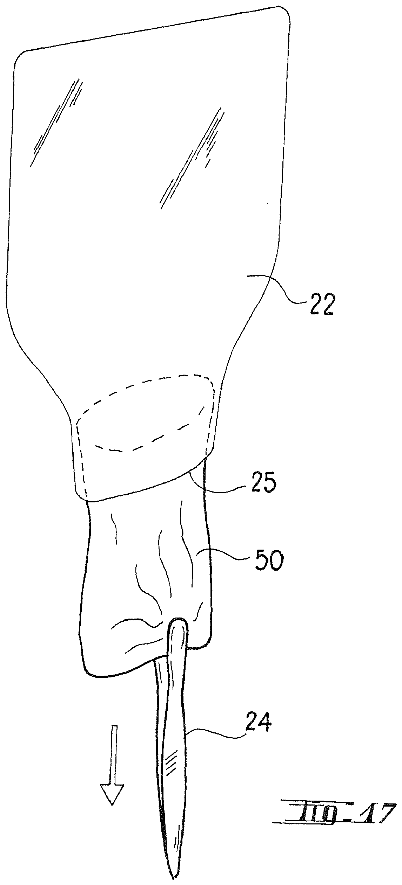

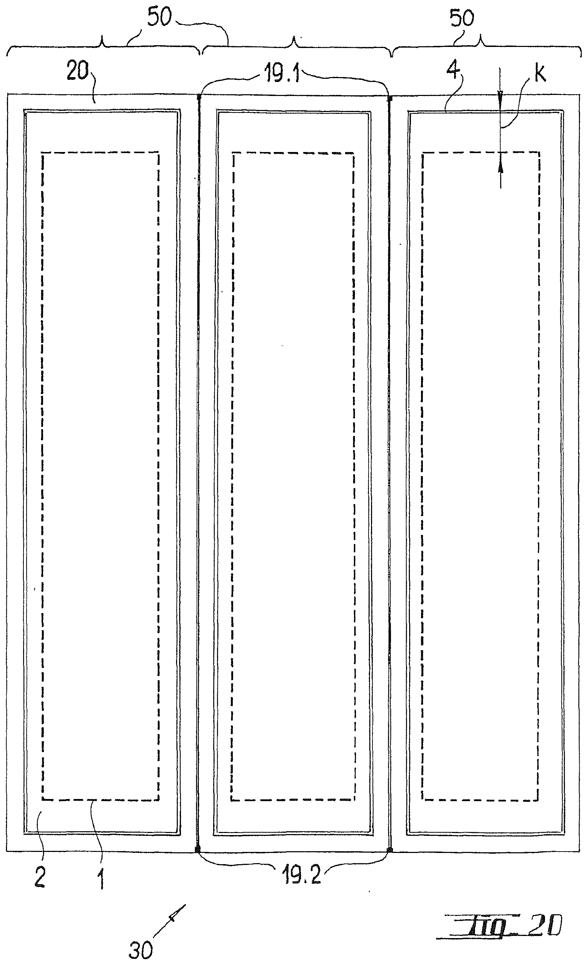

Weiterhin zeigt die Fig. 20 ein Ensemble 30, bestehend aus drei flachen, rechteckigen Absorptionskörpern 50, die jeweils etwa 20 cm lang und etwa 3 cm bis 4 cm breit sind. Insgesamt ergibt sich ein Flachgebilde, das in einen marktüblichen Ileostomiebeutel eingelegt werden kann. Dadurch, dass die Absorptionskörper 50 miteinander nur an zwei punktförmigen Verbindungsstellen 19.1, 19.2 verschweißt sind, könnnen die einzelnen Absorptionskörper Stück für Stück mit einer Pinzette 24 über eine vorhandene Öffnung gegriffen, abgetrennt und herausgenommen werden. Hierbei ist ein ausreichender freier Abstand k zwischen dem Materialabschnitt 1 und der Naht 4 gewährleistet, damit das Manipulieren mit der Pinzette erleichtert werden kann. Die Fig. 17 zeigt ein Iloestomiebeutel 22, aus dem ein verbrauchter Absorptionskörper 50 an dessen unterer Öffnung 25 mithilfe der Pinzette 24 herausgenommen wird.

Schließlich zeigt die Fig. 21 ein weiteres Ensemble 31, bestehend aus zwei miteinander über eine durchgehende Perforationslinie 32 verbundenen ebenso flachen, rechteckigen Absorptionskörpern 50. Die Perforationslinie 32 ermöglicht ein einfaches Trennen der beiden Absorptionskörper voneinander. Wie aus der Fig. 21 erkennbar, verläuft die Perforationslinie 32 genau in der Mitte zwischen den Nahtabschnitten der Absorptionskörper unter Beibehaltung der vorgesehenen Überstände 5. Dies ermöglicht dem Fachpersonal, das Produkt z. B. bis zur Mitte der Perforationslinie 32 einzureißen und um eine Schlauchleitung herumzulegen. Das Ensemble kann an der Perforationslinie 32 zusammengefaltet werden.

Der Absorptionskörper bzw. das Ensemble gemäß Erfindung wird vorzugsweise als sterilisiertes, gasdicht verpacktes Produkt vertrieben.

### Bezugszeichenliste:

- 1: Absorptionsmaterial
- 2: Hülle
- 3: Folienabschnitt
- 4: Naht
- 5: Überstand
- 6: Breite
- 7.1, 7.2: Außenkante
- 8: Klebepunkt
- 9: Umhüllung
- 10: Trennwand
- 11: Einschnitt
- 12: Zwischenlage
- 13: Außenseite
- 14: Körper
- 15: Wunde
- 16: Zwischenlage
- 17: Schnittlinie
- 18.1, 18.2: Flügel
- 19.1, 19.2: Verbindungsstelle
- 20: Poren
- 21: Öffnung
- 22: Iloestomiebeutel
- 23: Zwischenlage
- 24: Pinzette
- 25: Öffnung
- 26.1, 26.2: Lage
- 27: Kolostomiebeutel
- 28: Trapez-Basiskante
- 29: Verklebung
- 30: Ensemble
- 31: Ensemble
- 32: Perforationslinie
- 33: .Eakbereich
- 42: Wölbung
- 50......62: Absorptionskörper
- F1; F2: Fläche
- H: Höhenmaß
- k: Abstand
- X: Richtung

## Patentansprüche

1. Absorptionskörper (50...62) zum Anschluss an den menschlichen Körper, insbesondere zum Aufsaugen von Flüssigkeiten, welche aus menschlichen Körperstellen, wie Wunden, austreten, aufweisend:
• einen im wesentlichen flachen Materialabschnitt aus Absorptionsmaterial (1), bestehend aus einem aufsaugenden Vlies mit darin verteilten Superabsorber-Teilchen,
• und eine flüssigkeitsdurchlässige Hülle (2), die den Materialabschnitt umgibt und eine Barriere gegen feste Ausscheidungen bildet und die den Durchtritt von anderen ausgetretenen Substanzen zu dem innerhalb der Hülle (2) angeordneten Materialabschnitt aus Absorptionsmaterial (1) ermöglicht, wobei die Hülle Poren (20) oder Maschen aufweist, welche jeweils kleiner als die unbenetzten Superabsorber-teilchen sind und wenigstens teilweise von einer Naht (4) abgeschlossen ist,
**dadurch gekennzeichnet, dass**
• der Materialabschnitt in Draufsicht auf seine Flachseite eine Fläche (F1) aufweist,
• welche in seinem nicht benetzten Zustand um 20% bis 75% kleiner als die Fläche (F2) der flachgelegten Hülle (2) ist, wobei die Hülle (2) durch die umlaufende Naht (4) begrenzt ist, und in der Hülle (2) frei beweglich oder fixiert ist,
• wobei die Hülle (2) in Draufsicht auf seine Flachseite einen umlaufenden, über die Naht (4) hinausragenden Überstand (5) aufweist, der wenigstens 1 mm breit ist und der Absorptionskörper frei von harten, scharfen Kanten und Ecken ist.

2. Absorptionskörper nach Anspruch 1, **dadurch gekennzeichnet, dass** der Überstand (5) und/oder die Naht (4) in den Eckbereichen (33) der Hülle (2) abgerundet ist.

3. Absorptionskörper nach Anspruch 1, **dadurch gekennzeichnet, dass** die Poren oder Maschen 0,05 mm bis 1,0 mm, vorzugsweise 0,20 mm bis 0,50 mm groß sind.

4. Absorptionskörper nach Anspruch 1 und 3, **dadurch gekennzeichnet, dass** die Poren oder Maschen durch die Fäden- oder Faserabschnitte (41) begrenzt sind, welche im Schnitt durch die Hülle (2) etwa bogenförmig sind und mit ihren Bogen-Scheiteln (42) nach außen zeigen.

5. Absorptionskörper nach einem der vorhergehenden Ansprüche, **dadurch gekennzeichnet, dass** die Hülle (2) aus einem weichgriffigen Textilmaterial, beispielsweise aus einem voileartigen Vliesstoff, hergestellt ist.

6. Absorptionskörper nach einem der Ansprüche 1 bis 5, **dadurch gekennzeichnet, dass** dieser in Draufsicht auf seine Flachseite kreisrund oder elliptisch ist.

7. Absorptionskörper nach einem der vorhergehenden Ansprüche, **dadurch gekennzeichnet, dass** an wenigstens einer Außenseite (13) der perforierten Hülle (2) ganzflächig ein flüssigkeitsundurchlässiger Folienabschnitt (3) angebracht ist.

8. Absorptionskörper nach Anspruch 1 bis 7, lösbar verbunden mit wenigstens einem nebeneinander angeordneten, weiteren Absorptionskörper, wobei sich die miteinander verbundenen Absorptionskörper voneinander per Hand abtrennen lassen.

9. Absorptionskörper nach Anspruch 8, **dadurch gekennzeichnet, dass** dieser an wenigstens einer am Überstand (5) oder Naht (4) angeordneten, punktartigen Verbindungsstelle (19.1, 19.2) mit dem benachbarten Absorptionskörper verschweißt, zusammengeklebt oder -geheftet ist.

10. Absorptionskörper nach Anspruch 8, **dadurch gekennzeichnet, dass** dieser über eine Perforationslinie (32) mit dem benachbarten Absorptionskörper wenigstens teilweise abreißbar verbunden ist.

## Claims

1. Absorption body (50...62) for connection to the human body, in particular for absorbing liquids which exude from parts of the human body, such as wounds, comprising:
• a substantially planar material segment made of absorption material (1), consisting of an absorbent non-woven material comprising superabsorber particles distributed therein,
• and a liquid-permeable envelope (2), which surrounds the material segment, forms a barrier against solid excretions, and allows other exuded substances to pass into the material segment made of absorption material (1) arranged inside the envelope (2), the envelope comprising pores (20) or meshes which are each smaller than the non-wetted superabsorber particles and being closed by a seam (4) at least in part,
**characterised in that**
• the material segment comprises a surface (F1) in a plan view of its flat side,
• which, when not wetted, is 20% to 75% smaller than the surface (F2) of the flat envelope (2), wherein the envelope (2) is delimited by the peripheral seam (4), and is freely movable or fixed in the envelope (2),
• wherein the envelope (2) comprises, in a plan view of its flat side, a peripheral protrusion (5) projecting beyond the seam (4), which is at least 1 mm wide, and the absorption body is free of hard, sharp edges and corners.

2. Absorption body according to claim 1, **characterised in that** the protrusion (5) and/or the seam (4) are rounded in the corner regions (33) of the envelope (2).

3. Absorption body according to claim 1, **characterised in that** the pores or meshes are 0.05 mm to 1.0 mm large, preferably 0.20 mm bis 0.50 mm large.

4. Absorption body according to claims 1 and 3, **characterised in that** the pores or meshes are delimited by the thread or fibre sections (41), which are approximately curved in the section through the envelope (2) and point outwards by the curve vertices (42) thereof.

5. Absorption body according to any of the preceding claims, **characterised in that** the envelope (2) is made of a soft textile material, for example of a voile-like non-woven fabric.

6. Absorption body according to any of claims 1 to 5, **characterised in that** said body is circular or elliptical in a plan view of its flat side.

7. Absorption body according to any of the preceding claims, **characterised in that** a liquid-impermeable film section (3) is attached to at least one outer face (13) of the perforated envelope (2) over the entire surface thereof.

8. Absorption body according to claim 1 to 7, which is detachably connected to at least one additional absorption body arranged therebeside, it being possible to separate the interconnected absorption bodies from one another by hand.

9. Absorption body according to claim 8, **characterised in that** said body is welded, adhered or crimped to the adjacent absorption body at at least one point-like connection point (19.1, 19.2) arranged on the protrusion (5) or seam (4).

10. Absorption body according to claim 8, **characterised in that** said body is connected to the adjacent absorption body by a perforation line (32) such that it can be torn off at least in part.

## Revendications

1. Corps d'absorption (50...62) destiné au raccordement au corps humain, en particulier destiné à l'absorption de liquides qui s'écoulent de zones du corps humain telles que les plaies, comportant :
• une portion de matériau sensiblement plate en matériau absorbant (1), constituée d'un nontissé absorbant avec des particules de polymère superabsorbant réparties dans celui-ci,
• et une enveloppe (2) perméable aux liquides, qui entoure la portion de matériau et forme une barrière pour les excrétions solides et qui permet le passage d'autres substances qui se sont écoulées vers la portion de matériau en matériau absorbant (1) disposée à l'intérieur de l'enveloppe (2), l'enveloppe comportant des pores (20) ou des mailles, qui sont respectivement plus petites que les particules de polymère superabsorbant non mouillées, et est au moins en partie fermée par une couture (4),
**caractérisé en ce que**
• la portion de matériau comporte, en vue de dessus sur son côté plat, une surface (F1),
• qui, dans l'état non mouillé de la portion de matériau, est inférieure de 20 % à 75 % à la surface (F2) de l'enveloppe (2) posée à plat, l'enveloppe (2) étant limitée par la couture (4) périphérique, et peut bouger librement ou est fixée dans l'enveloppe (2),
• l'enveloppe (2) comportant, en vue de dessus sur son côté plat, un dépassement (5) périphérique dépassant la couture (4), qui est large d'au moins 1 mm et le corps d'absorption étant dénué de bords et coins vifs et pointus.

2. Corps d'absorption selon la revendication 1, **caractérisé en ce que** le dépassement (5) et/ou la couture (4) sont arrondis dans les zones de coin (33) de l'enveloppe (2).

3. Corps d'absorption selon la revendication 1, **caractérisé en ce que** les pores ou mailles mesurent de 0,05 mm à 1,0 mm, de préférence de 0,20 mm à 0,50 mm.

4. Corps d'absorption selon les revendications 1 et 3, **caractérisé en ce que** les pores ou mailles sont limités par les portions de fils ou de fibres (41), qui, en coupe à travers l'enveloppe (2), sont approximativement arquées et qui sont orientées avec leurs sommets d'arc (42) vers l'extérieur.

5. Corps d'absorption selon l'une des revendications précédentes, **caractérisé en ce que** l'enveloppe (2) est fabriquée en un matériau textile au toucher doux, par exemple en nontissé de type voilage.

6. Corps d'absorption selon l'une des revendications 1 à 5, **caractérisé en ce qu'**il est, en vue de dessus sur son côté plat, circulaire ou elliptique.

7. Corps d'absorption selon l'une des revendications précédentes, **caractérisé en ce que**, sur au moins un côté extérieur (13) de l'enveloppe (2) perforée, une portion de film (3) imperméable aux liquides est appliquée sur toute la surface.

8. Corps d'absorption selon les revendications 1 à 7, relié de manière détachable à au moins un autre corps d'absorption disposé à côté de lui, les corps d'absorption reliés l'un à l'autre pouvant être séparés à la main.

9. Corps d'absorption selon la revendication 8, **caractérisé en ce qu'**il est soudé, collé ou agrafé au corps d'absorption adjacent en au moins un emplacement de liaison (19.1, 19.2) ponctuel situé sur le dépassement (5) ou la couture (4).

10. Corps d'absorption selon la revendication 8, **caractérisé en ce qu'**il est relié au corps d'absorption adjacent de manière détachable au moins en partie par le biais d'une ligne de perforation (32).
